(19) **Europäisches Patentamt**
**European Patent Office**
**Office européen des brevets**

(11) **EP 2 924 092 A1**

(12) **EUROPEAN PATENT APPLICATION**
published in accordance with Art. 153(4) EPC

(43) Date of publication:
**30.09.2015 Bulletin 2015/40**

(21) Application number: **13856765.6**

(22) Date of filing: **20.11.2013**

(51) Int Cl.:
*C09J 201/00* (2006.01)     *A61K 9/70* (2006.01)
*C09J 4/02* (2006.01)     *C09J 7/02* (2006.01)
*C09J 11/06* (2006.01)     *C09J 11/08* (2006.01)

(86) International application number:
**PCT/JP2013/081316**

(87) International publication number:
**WO 2014/080954 (30.05.2014 Gazette 2014/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **21.11.2012 JP 2012255689**

(71) Applicant: **Alcare Co., Ltd.**
**Sumida-ku**
**Tokyo 131-0046 (JP)**

(72) Inventors:
- **WATANABE Ryota**
  **Tokyo 131-0046 (JP)**
- **OKUYAMA Wataru**
  **Tokyo 131-0046 (JP)**

(74) Representative: **D Young & Co LLP**
**120 Holborn**
**London EC1N 2DY (GB)**

(54) **ADHESIVE COMPOSITION FOR SKIN, ADHESIVE FOR SKIN, AND ADHESIVE SHEET FOR SKIN**

(57)     An object of the invention is to provide an adhesive composition for skin that gives an adhesive for skin that can be coated readily and is highly cohesive and also an adhesive for skin and an adhesive sheet for skin prepared by using the adhesive composition for skin.

Provided is an adhesive composition for skin including a radiation-curable resin and a reactive diluent that reacts with the radiation-curable resin. The adhesive composition for skin gives an adhesive for skin that can be coated readily and is highly cohesive. Also provided are an adhesive for skin prepared by irradiating the adhesive composition for skin with radiation and an adhesive sheet for skin including a base sheet and an adhesive layer of the composition for skin formed thereon.

**Description**

Technical Field

The present invention relates to an adhesive composition for skin and also to an adhesive for skin and an adhesive sheet for skin obtained from the adhesive composition for skin. More specifically, it relates, for example, to an adhesive composition for skin that gives an adhesive readily processable (e.g., coatable) and highly cohesive.

Background Art

Adhesive patches have been used to fix medical materials and devices such as gauzes, bandages, and catheters to the skin and also, for example, for protection and treatment of skin and skin wounds.
Acrylic and rubber-based adhesives have been used frequently as the adhesive for adhesive patches. It is reported recently that adhesives of a radiation-curable resin have been used, replacing the acrylic and rubber-based adhesives.

Citation List

Patent Literatures

[Patent Document 1] JP-T No. 2000-503009

Summary of Invention

Technical Problem

However, such an adhesive patch has a problem that it is easily separated from the skin, for example by perspiration and transpiration, when it is adhered for an extended period of time. It also has problems that the cohesive force thereof declines when a plasticizer is added to the adhesive patch in a great amount to make it more flexible and expandable to cope with the irregularity of the surface of the skin and the extension and shrinkage of the skin, and the adhesive remains deposited on the skin when the adhesive patch is removed after application for an extended period of time.
Additives are often added to the adhesive patch to provide it with a new functionality. However, addition of additives generally leads to increase in viscosity of the adhesive, making the additives less miscible and coating operation more difficult.
Accordingly, an object of the present invention, which was made under the circumstances above, is to provide an adhesive composition for skin that gives an adhesive for skin that can be coated readily and is highly cohesive.

Solution to Problem

After intensive studies under the circumstances above, the inventors have found that it is possible to produce an adhesive composition for skin that gives an adhesive for skin that can be coated readily and is highly cohesive by using a radiation-curable resin that cures after application of the adhesive composition for skin and a reactive diluent that reacts therewith and made the present invention.
Specifically, the present invention provides an adhesive composition for skin including a radiation-curable resin and a reactive diluent that reacts with the radiation-curable resin.
The radiation-curable resin for use may be a UV-curable resin.
The reactive diluent may contain a (meth)acryloyl group-containing compound.
The adhesive composition for skin according to the present invention may include a UV-curable resin as the radiation-curable resin in an amount of 30 to 95 mass % and a (meth)acryloyl group-containing reactive diluent as the reactive diluent in an amount of 1 to 30 mass %.
The reactive diluent for use may be a reactive diluent having 1 to 3 (meth)acryloyl groups in the molecule or a trimethylolpropane skeleton-containing (meth)acrylate.
The adhesive composition for skin according to the present invention may further include a hydrophilic polymer.
The adhesive composition for skin may include a UV-curable resin as the radiation-curable resin in an amount of 30 to 90 mass %, the reactive diluent in an amount of 1 to 30 mass %, and a hydrophilic polymer in an amount of 5 to 20 mass %.
The adhesive composition for skin according to the present invention may further include a functional group-free acrylic polymer and it may include the functional group-free acrylic polymer in an amount of 1 to 20 mass %.

In addition, the present invention provides an adhesive for skin prepared by irradiating the adhesive composition for skin with radiation.

Further, the present invention provides an adhesive sheet for skin including a base sheet and an adhesive layer formed thereon that is prepared by irradiating the adhesive composition for skin with radiation.

Advantageous Effects of Invention

It is possible according to the present invention to provide an adhesive composition for skin that gives an adhesive for skin that can be coated readily and is highly cohesive.

Description of Embodiments

Hereinafter, favorable embodiments of the present invention will be described. The embodiments described below are some typical embodiments of the present invention and it should be understood that the scope of the present invention shall not be restricted thereby.

<Adhesive composition for skin>

The adhesive composition for skin in the present embodiment of the invention includes, for example, a radiation-curable resin and a reactive diluent that reacts with the radiation-curable resin.

Alternatively, the adhesive composition for skin in the present embodiment of the invention includes, for example, a radiation-curable resin, a hydrophilic polymer, and a reactive diluent that reacts with the radiation-curable resin.

The adhesive composition for skin according to the present invention is a fluidal liquid before irradiation with radiation that causes curing of the radiation-curable resin contained in the adhesive composition for skin.

In the present invention, the adhesive composition for skin after irradiation with radiation will be called an "adhesive for skin," to differentiate it from the adhesive composition for skin before irradiation with radiation.

When the adhesive composition for skin contains a hydrophilic polymer, the state of the radiation-curable resin, the hydrophilic polymer, and the reactive diluent is not particularly limited, but the adhesive composition is preferably in the hydrocolloidal state in which the hydrophilic polymer is dispersed in the radiation-curable resin (and also in the reactive diluent reacting therewith). The hydrocolloidal adhesive composition for skin can absorb, for example, sweat and wound exudate, alleviating the skin eruption and itchiness by stuffiness and thus can be used favorably as a wound dressing.

The "radiation-curable resin" contained in the adhesive composition for skin of the present embodiment is a resin composition that cures (or crosslinks) by irradiation with radiation and contains at least one of polymers, oligomers, and monomers. For example, the radiation-curable resin for use may be a polymerization product from a monomer and a polymerization initiator or alternatively, an oligomer or a monomer that cures (or crosslinks) by irradiation with radiation.

The "radiation" irradiated to the radiation-curable resin is not particularly limited, if it can provide the adhesive composition for skin with an energy needed for causing a curing (crosslinking) reaction (e.g., radical, cationic, or anionic polymerization) by irradiation.

Examples of the radiations include electron beams, ultraviolet rays, infrared rays, laser beams, visible rays, ionizing radiations (X-, $\alpha$-, $\beta$-, $\gamma$-rays etc.), microwaves, high-frequency waves, and the like.

The radiation-curable resin for use in the embodiment is preferably a resin that cures (crosslinks) by one or more radiation selected from the group consisting of electron beams, visible rays, and ultraviolet rays, among the radiation species above.

The radiation-curable resin is preferably one or more resins selected from electron beam-, visible ray-, and UV-curable resins. Among the resins above, a UV-curable resin is used more preferably.

The mechanism of the curing reaction of the radiation-curable resin is not particularly limited, but radical, cationic, or photodimerization reaction is preferable, and radical or photodimerization reaction is more preferable.

Examples of the radiation-curable resins include (meth)acrylate resins, silicone resins, urethane resins, silicone acrylate-based resins, urethane acrylate-based resins, epoxy acrylate-based resins, and the like. These radiation-curable resins may be used alone or in combination of two or more. They may be used as a mixture of two or more.

Among the resins above, the radiation-curable resin is preferably one or more resins selected from (meth)acrylate resins, silicone (meth)acrylate resins, urethane (meth)acrylate resins, and epoxy (meth)acrylate resins. The radiation-curable resin for use is more preferably a UV-curing (meth)acrylate resin.

The "(meth)acrylate," as used in the present embodiment, means both "acrylate" and "methacrylate."

The radiation-curable resin for use is preferably a polymer and/or a copolymer having a (meth)acrylate-derived structural unit (recurring unit) containing an alkyl group having 1 to 20 carbon atoms (more preferably 2 to 10 and still more preferably 4 to 8 carbon atoms) or a cycloalkyl group having 4 to 8 carbon atoms. The alkyl or cycloalkyl group may have one or more substituent groups and examples of the substituent groups include halogen atoms, a hydroxy group, aryl groups, alkoxy groups, phenoxy groups, an epoxy group, a norbornyl group, an adamantyl group, and the like.

The radiation-curable resin having such a structural unit is more preferably a polymer and/or a copolymer having a butyl (meth)acrylate-derived structural unit and/or a 2-ethylhexyl (meth)acrylate-derived structural unit, still more preferably a polymer and/or a copolymer having a butyl acrylate-derived structural unit and/or a 2-ethylhexyl acrylate-derived structural unit.

The polymer and/or copolymer having such a structural unit may be an oligomer having a smaller molecular weight than polymers, which is described below.

The mass-average molecular weight Mw of the radiation-curable resin is preferably 1000 to 250000 and more preferably 1000 to 200000. When the Mw of the radiation-curable resin is in the range above, it is possible to obtain a low-viscosity adhesive composition for skin and a highly cohesive adhesive with having a mass-average molecular weight increased by curing reaction after irradiation with radiation. The Mw is a value in terms of polystyrene, as determined by gel-permeation chromatography (GPC).

The radiation-curable resin for use may be a commercially available product.

Examples of the commercial products of radiation-curable resins include "3000 series" and "3100 series" products manufactured by ThreeBond Holdings Co., Ltd.; "UNIDIC" and "TYFORCE" series products manufactured by DIC Corporation; "Yupimer" series products manufactured by Mitsubishi Chemical Corporation; "HITALOID" series products manufactured by Hitachi Chemical Co., Ltd.; "BEAMSET" series products manufactured by Arakawa Chemical Industries, Ltd.; "acResin" series products manufactured by BASF; "UVA-2000 series" products manufactured by Toagosei Co., Ltd.; and the like.

The radiation-curable resin may be a resin composition including one or more polymerizable oligomers and/or monomers as components, in addition to or in place of the polymer and/or copolymer having a (meth)acrylate-derived structural unit described above. The mechanism of polymerization of the polymerizable oligomers and/or monomers is preferably radical or cationic polymerization and more preferably radical polymerization.

Examples of the radically polymerizable oligomers include (meth)acrylate-based oligomers, polyester acrylate-based oligomers, urethane (meth)acrylate-based oligomers, silicone (meth)acrylate-based oligomers, epoxy (meth)acrylate-based oligomers, and the like.

Examples of the radically polymerizable monomers include (meth)acryloyl group-containing monomers. The number of the (meth)acryloyl groups of each monomer is not particularly limited, and examples of such monomers include monofunctional (meth)acrylates having one (meth)acryloyl group, bifunctional (meth)acrylates having two (meth)acryloyl groups, and multifunctional (meth)acrylates having three or more (meth)acryloyl groups.

Examples of the cationically polymerizable oligomers or monomers include oligomers or monomers containing an epoxy ring, an oxetane ring, an oxolane ring, a dioxolane ring, or a vinyl ether structure, and those containing a functional group having such a structure.

Typical examples of the cationically polymerizable oligomer or monomers include limonene oxide compounds commercially available from Tomoe Engineering Co., Ltd., glycidyl ether compounds which are commercially available under the trade name of "Epolite" manufactured by Kyoeisha Chemical Co., Ltd., oxetane compounds commercially available under the trade name of "Aron Oxetane" manufactured by Toagosei Co., Ltd., and the like.

The radiation-curable resin used in the present embodiment preferably contains, as the polymerizable oligomer above, one or more oligomers selected from (meth)acrylate-, urethane (meth)acrylate-, and silicone (meth)acrylate-based oligomers.

The molecular weight of the oligomer above is not particularly limited, but, for example, an oligomer having a number-average molecular weight of less than 10000 (e.g., 1000 or more and less than 10000) can be used. An oligomer having a number-average molecular weight of less than 10000 is preferable, as it can give an adhesive composition for skin not so excessively viscous that it can be processed (for example coated) favorably by use of a reactive diluent described below. It is also possible to use a high-molecular-weight polymerizable oligomer having a number-average molecular weight of about 10000 or more. The number-average molecular weight Mn is a value in terms of polystyrene, as determined by gel-permeation chromatography (GPC).

The radiation-curable resin above may contain a polymerization initiator, an additive, a solvent, and others.

The polymerization initiator is not particularly limited, and any polymerization initiator known in the field of radiation-curable resin may be used. Examples thereof include acetophenone-based initiators, benzophenone compounds, $\alpha$-ketoester compounds, benzoin compounds, and the like.

It is preferable that a polymerization initiator is bound to the molecular chain of the polymer or oligomer, a component of the radiation-curable resin, instead of the polymerization initiator being contained in the radiation-curable resin. Such a radiation-curable resin containing a polymerization initiator bound to the molecular chain, if used, eliminates the need for addition of a polymerization initiator and yet gives a safer adhesive composition for skin.

Examples of the radiation-curable resins containing a polymerization initiator bound to the molecular chain include "UV-H" series products manufactured by KSM Co., Ltd., "acResin" series products manufactured by BASF, and the like.

Another polymerization initiator may be added to the radiation-curable resin containing a polymerization initiator previously bound thereto or another polymerization initiator may be added to replace the existing polymerization initiator.

Examples of the photodimerizable oligomers or polymers include maleimide group-containing oligomers or polymers and the like.

Examples of the photodimerizable oligomers or polymers include maleimide-terminated polyester resins or maleimide-terminated polyether resins commercially available under the trade name of "UVA-2000" series manufactured by Toagosei Co., Ltd., and the like.

The content of the radiation-curable resin used in the present embodiment is not particularly limited, but preferably 30 to 95 mass %, more preferably 30 to 90 mass % and still more preferably 40 to 80 mass %, in the total mass of the adhesive composition for skin.

When the content of the radiation-curable resin is in the range above, it is possible to make the adhesive for skin obtained after irradiating the adhesive composition for skin with radiation exhibit favorable cohesiveness. It is also possible to form easily a hydrocolloid in which the hydrophilic polymer described below is dispersed in the radiation-curable resin.

The adhesive composition for skin of the present embodiment contains a reactive diluent that reacts with the radiation-curable resin. The "reactive diluent," as used herein, is a liquid that can react with the radiation-curable resin and reduce the viscosity of the adhesive composition for skin or a liquid that can reduce the amount of resin components present in the adhesive composition for skin.

As the adhesive composition for skin of the present embodiment contain a reactive diluent, for example, the adhesive composition can be processed (for example coated) easily and, as the reactive diluent reacts and crosslinks with the radiation-curable resin after irradiation with radiation, an adhesive superior in cohesiveness and resistant to bleeding can be obtained.

The reactive diluent is preferably a compound containing a (meth)acryloyl group as a functional group. The "(meth)acryloyl group," as described herein, means both an "acryloyl group" ($H_2C=CH-C(=O)-$) and a "methacryloyl group" ($H_2C=C(CH_3)-C(=O)-$).

The reactive diluent used in the present embodiment may be added to a commercially available radiation-curable resin.

The number of the (meth)acryloyl groups in the (meth)acryloyl group-containing compound is not particularly limited, but preferably 1 to 3, and thus, the reactive diluent preferably has 1 to 3 (meth)acryloyl groups in the molecule. The molecular weight of the (meth)acryloyl group-containing compound is not also particularly limited, but preferably 1000 or less, more preferably 800 or less and still more preferably 600 or less.

Examples of the (meth)acryloyl group-containing compounds used as the reactive diluent include (meth)acrylate compounds, (meth)acrylamide compounds, and the like.

The "(meth)acrylate compound," as described herein, is a (meth)acrylic ester having a straight- or branched-chain alkyl group or a cycloalkyl group, wherein the alkyl or cycloalkyl group may have one or more substituents.

The "(meth)acrylamide compound" is a (meth)acryloyl group-containing amide compound, wherein the amino group of the (meth)acrylamide may have one or more substituents. The term "(meth)acrylic" means both "acrylic" and "methacrylic."

The substituent group in the "(meth)acrylate compounds" and "(meth)acrylamide compounds" is not particularly limited and may be, for example, a hydroxyl group, an alkoxy group, a phenoxy group, or the like.

Examples of the (meth)acrylate compounds include monofunctional (meth)acrylate compounds having one (meth)acryloyl group, bifunctional (meth)acrylate compound having two (meth)acryloyl groups, and multifunctional (meth)acrylate compounds having 3 or more (meth)acryloyl groups.

Examples of the monofunctional (meth)acrylate compounds include methyl (meth)acrylate, ethyl (meth)acrylate, n-propyl (meth)acrylate, isopropyl (meth)acrylate, n-butyl (meth)acrylate, isobutyl (meth)acrylate, t-butyl (meth)acrylate, isooctyl (meth)acrylate, 2-ethylhexyl (meth)acrylate, isodecyl (meth)acrylate, tridecyl (meth)acrylate, isoamyl (meth)acrylate, stearyl (meth)acrylate, lauryl (meth)acrylate, cyclohexyl (meth)acrylate, isobornyl (meth)acrylate, 2-hydroxyethyl (meth)acrylate, 2-hydroxypropyl (meth)acrylate, 2-hydroxybutyl (meth)acrylate, ethoxydiethylene glycol (meth)acrylate, methoxydiethylene glycol (meth)acrylate, methoxypolyethylene glycol (meth)acrylate, phenoxyethyl (meth)acrylate, 2-hydroxy-3-phenoxypropyl (meth)acrylate, cyclohexane spiro-2-(1,3-dioxolan-4-yl))methyl acrylate, and the like.

Examples of the bifunctional (meth)acrylate compounds include ethylene glycol di(meth)acrylate, diethylene glycol di(meth)acrylate, triethylene glycol di(meth)acrylate, tetraethylene glycol di(meth)acrylate, polyethylene glycol di(meth)acrylate, dipropylene glycol dimethacrylate, tripropylene glycol di(meth)acrylate, 1,3-butylene glycol di(meth)acrylate, 1,4-butanediol di(meth)acrylate, 1,6-hexanediol di(meth)acrylate, 1,9-nonanediol di(meth)acrylate, and the like.

Examples of the trifunctional or higher multifunctional (meth)acrylate compounds include trimethylolpropane tri(meth)acrylate, alkylene oxide-modified trimethylolpropane tri(meth)acrylates, pentaerythritol tri(meth)acrylate, pentaerythritol tetra(meth)acrylate, ditrimethyrollpropane tetra(meth)acrylate, dipentaerythritol penta(meth)acrylate, dipentaerythritol hexa(meth)acrylate, and the like.

The term "alkylene oxide-modified" in the "alkylene oxide-modified trimethylolpropane tri(meth)acrylate" is, for example, ethylene oxide (EO)-modified or propylene oxide (PO)-modified.

Among the (meth)acrylate compounds above, the number of the "glycol" constituent units in the compound having "ethylene glycol" or "propylene glycol" constituent units may be 1, 2, 3, 4 or more (the term "more" means a constituent unit number of 5 or more).

The (meth)acrylamide compound is, for example, an N-substituted (meth)acrylamide and examples thereof include N-methyl (meth)acrylamide, N-ethyl (meth)acrylamide, N-(meth)acryloylmorpholine, N,N-dimethyl (meth)acrylamide, N,N-diethyl (meth)acrylamide, N,N-dimethylaminopropyl (meth)acrylamide, N-hydroxyethyl (meth)acrylamide, N-methyl-N-hydroxyethyl (meth)acrylamide, N-hydroxypropyl (meth)acrylamide, N,N-bishydroxyethyl (meth)acrylamide, and the like.

The reactive diluent for use may be one or more monomers in the (meth)acryloyl group-containing compounds above or an oligomer containing the monomers as constituent monomers.

The reactive diluent for use may be a dendrimer-type (meth)acrylate having a molecular structure in which the polymer chains are branched orderly from the center.

The "(meth)acryloyl group-containing compound" used as the reactive diluent is preferably one or more compounds selected from acryloylmorpholine, hydroxyethylacrylamide, phenoxyethyl acrylate, 1,3-dioxolane-based acrylates, isobornyl acrylate, polyethylene glycol diacrylate, PO-modified trimethylolpropane triacrylates, and methoxypolyethylene glycol acrylate.

It is more preferably one or more compounds selected from acryloylmorpholine, polyethylene glycol diacrylate, and PO-modified trimethylolpropane triacrylates. It is still more preferably a trifunctional or higher multifunctional compound.

As the above mentioned (meth)acrylate compound that can be used as the reactive diluent, it is also favorable to use a trimethylolpropane skeleton-containing (meth)acrylate that has a quaternary carbon atom and shows symmetry on the quaternary carbon. Examples of the trimethylolpropane skeleton-containing (meth)acrylate include trimethylolpropane tri(meth)acrylate, ditrimethyrollpropane tetra(meth)acrylate, alkylene oxide-modified trimethylolpropane tri(meth)acrylates, and the like, as described above.

The reactive diluent is preferably less irritant to the skin. The primary irritation index PII of the reactive diluent, as determined by primary skin irritation test, is preferably 0.0 to 1.9, more preferably 0.0 to 1.0, and still more preferably 0.0 to 0.4.

When the PII of the reactive diluent is in the range above, it is possible to obtain an adhesive composition for skin less stimulating to the skin and having high safety. The primary skin irritation test can be performed, for example, according to the test method specified in the "OECD Guidelines for the Testing of Chemicals 404 (2002)."

The reactive diluent preferably has a viscosity at 25°C of 1000 mPa•s or less and more preferably 500 mPa•s or less and the lower limit is preferably 1 mPa•s or more.

When viscosity of the reactive diluent at 25°C is in the range above, it is possible to mix the reactive diluent easily with the radiation-curable resin and make the processing, such as coating, easier. In the present description, the viscosity of the reactive diluent at 25°C (steady-flow viscosity) is a value determined according to JIS Z8803 at 25°C, with a single-cylinder rotating viscometer.

The content of the reactive diluent used in the present embodiment is not particularly limited, but preferably 1 to 30 mass % in the total mass of the adhesive composition for skin. For effective reduction of the viscosity of the adhesive composition for skin and for production of an adhesive for skin with high adhesive strength, the content of the reactive diluent is preferably 1 to 20 mass %, more preferably 2 to 15 mass %, and still more preferably 2 to 8 mass % in the total mass of the adhesive composition for skin. The total content of the radiation-irradiation resin and the reactive diluent is also preferably 50 to 100 mass %, more preferably 60 to 85 mass %, and still more preferably 70 to 85 mass % in the total mass of the adhesive composition for skin.

When the content of the reactive diluent is in the range above, it is possible to reduce the viscosity of the adhesive composition for skin and make processing (such as coating) thereof easier. It is also possible to obtain easily an adhesive for skin resistant to bleeding and higher in cohesiveness.

The hydrophilic polymer contained in the adhesive composition for skin in another embodiment of the invention is not particularly limited and may be a natural, semisynthetic, or synthetic hydrophilic polymer. The term "semisynthetic," which is also called partially chemical synthetic, means chemical synthesis in which, for example, a compound isolated from natural resources such as plants, microbes, and cell cultures is used as the starting material.

Typical examples of the natural hydrophilic polymers include plant polymers such as gum arabic, tragacanth gum, galactan, guar gum, carob gum, karaya gum, carrageenan, pectin, agar, and starch (e.g., rice, corn, potato, and wheat starches); microbial polymers such as xanthan gum, dextrin, dextran, succinoglucan, mannan, locust bean gum, and pullulan; animal polymers such as casein, albumin, and gelatin; and the like.

Typical examples of the semisynthetic hydrophilic polymer compounds include starch-based polymers (e.g., carboxymethylstarch, methylhydroxypropylstarch, etc.), cellulosic polymers (e.g., methylcellulose, ethylcellulose, methylhydroxypropylcellulose hydroxyethylcellulose, sodium cellulosesulfate, hydroxypropylcellulose, carboxymethylcellulose, sodium carboxymethylcellulose, etc.), alginic acid-based polymers (e.g., sodium alginate, alginic acid propylene glycol ester, etc.), and the like.

Typical examples of the synthetic hydrophilic polymer compounds include vinyl-based polymers (e.g., polyvinylalcohol, polyvinylmethylether, polyvinylpyrrolidone, carboxyvinylpolymer, etc.), acrylic polymers (e.g., sodium polyacrylate, polyacrylamide, etc.), polyethyleneimine, and the like.

The hydrophilic polymers may be used alone or in combination of two or more.

Among the hydrophilic polymers above, it is preferable to use one or more polymers selected from sodium carboxymethylcellulose, pectin, karaya gum, mannan, locust bean gum, and gelatin, and more preferable to use one or more polymers selected from sodium carboxymethylcellulose, mannan, and locust bean gum.

When the adhesive composition for skin contains these hydrophilic polymers, it is possible to form a so-called hydrocolloid easily. When the adhesive composition for skin forms a hydrocolloid, the hydrocolloidal adhesive composition for skin can absorb for example sweat and wound exudate, alleviating skin eruption and itchiness by stuffiness.

The content of the hydrophilic polymer is preferably 5 to 30 mass % and more preferably 5 to 20 mass %, in the total mass of the adhesive composition for skin.

When the content of the hydrophilic polymer is in the range above, it is possible to obtain an adhesive for skin that can absorb water such as sweat and wound exudate favorably. It is also possible to alleviate skin irritation, for example by skin maceration, and make the adhesive for skin adhered to the skin for an extended period of time.

The adhesive composition for skin of the present embodiment may contain, as needed, a tackifier, a filler, a pH adjuster, a pharmaceutical component, a softener (plasticizer), and others in the range that does not impair the object of the present invention.

The tackifier that can be used in the adhesive composition for skin of the present embodiment is not limited in kind, if the object of the present invention is not impaired, and any known tackifier may be used. Examples of the tackifiers include rosin derivatives, terpene resins, coumarone-indene resins, petroleum-based hydrocarbon resins, and the like.

When the adhesive composition for skin contains a tackifier, the content thereof is preferably 1 to 20 mass % and more preferably 5 to 15 mass %, in the total mass of the adhesive composition for skin.

When the content of the tackifier is in the range above, it is possible to provide an adhesive for skin with suitable adhesive strength.

The filler that can be used in the adhesive composition for skin of the present embodiment is not particularly limited in kind, if it does not impair the object of the present invention, and any known filler may be used. Examples of the fillers include silica, alumina, talc, and the like. These fillers are added, for example, to provide the adhesive composition for skin with thixotropy.

The pH adjuster that can be used in the adhesive composition for skin of the present embodiment is not particularly limited in kind, if it does not impair the object of the present invention, and any known pH adjuster may be used. Examples of the pH adjusters include citric anhydride, alkali metal hydroxides, buffered organic acid solutions, and the like.

The pharmaceutical component that can be used in the adhesive composition for skin of the present embodiment is not particularly limited in kind, if it does not impair the object of the present invention. Examples of the pharmaceutical components include drugs such as physiologically active substances, antibacterial agents, anti-inflammatory analgesic agents, steroids, anesthesias, antifungal drugs, bronchodilators, antitussives, coronary vasodilators, anti-hypertensive agents, hypotensive diuretics, antihistaminics, hypnotics and sedatives, tranquilizers, vitamin compounds, sex hormone preparations, antidepressants, cerebral ameliorators, antiemetic drugs, and antitumor drugs. These drugs show their action systemically or locally by percutaneous absorption or locally in the patched area.

The adhesive composition for skin of the present embodiment preferably does not contain any softener, but may contain a known softener in the range that does not impair the object of the present invention. Examples of the softeners include mineral oils such as paraffins and naphthene oils; vegetable oils such as olive oil, castor oil, and palm oil; animal oils such as lanolin, turtle oil, and beeswax; synthetic oils such as silicone- and ester-based oils; and the like.

As the adhesive composition for skin of the present embodiment contains a reactive diluent, it can be processed, for example coated, easily even when it contains no softener.

When the adhesive composition for skin contains a softener, the softener component may bleed out. After intensive studies by the inventors, it was found that the bleeding readily occurs when the softener component is contained in an amount of 20 mass % or more in the total mass of the adhesive composition for skin.

Thus in the present embodiment, an adhesive for skin resistant to bleeding can be obtained by preparing an adhesive composition in a readily coatable low-viscosity state, using a radiation-curable resin as a base material and a reactive diluent that reacts with the resin and making the reactive diluent bound to the radiation-curable resin by irradiation with radiation.

As the adhesive composition for skin of the present embodiment contains a reactive diluent, the curing reaction proceeds more easily and, as a result, it is possible to reduce the irradiation amount of the radiation needed for curing, as compared to the adhesive composition containing only a softener but no reactive diluent. Thus, it is also possible to improve productivity.

The adhesive composition for skin may contain additionally a functional group-free acrylic polymer as a component for reducing the viscosity of the adhesive composition for skin.

When the adhesive composition for skin does not contain any reactive diluent and contains a viscosity-lowering component such as a softener (plasticizer), the adhesive composition may bleed out, leading to deterioration of the cohesiveness of the resulting adhesive. However after further studies, the inventors have found unexpectedly that it is possible to reduce the viscosity of the adhesive composition for skin effectively and to increase the adhesive strength of the adhesive for skin by using a reactive diluent and a functional group-free acrylic polymer simultaneously.

Specifically, the adhesive composition for skin in yet another embodiment of the present invention includes a radiation-curable resin, a reactive diluent that reacts with the radiation-curable resin, and a functional group-free acrylic polymer. The adhesive composition for skin preferably contains the hydrophilic polymer described above and may contain additionally a tackifier, a filler, a pH adjuster, a pharmaceutical component, a softener (plasticizer), and others described above.

The "acrylic polymer" in the functional group-free acrylic polymer is a polyacrylate or a polymethacrylate, i.e., a polymer or a copolymer containing acrylate and/or methacrylate-derived structural units as the major structural units in an amount of 50 mol % or more. The acrylic polymer preferably contains the acrylate and/or methacrylate-derived structural units in an amount of 60 to 100 mol %, more preferably 70 to 100 mol %, and still more preferably 80 to 100 mol %. The content (mol %) of the acrylate and/or methacrylate-derived structural units in the acrylic polymer can be determined by NMR (Nuclear Magnetic Resonance).

The term "functional group-free" in the functional group-free acrylic polymer indicates that there are substantially no other functional groups than acryloyl groups. Examples of the other functional groups then include OH, COOH, an epoxy group, alkoxysilyl groups, and the like.

The mass-average molecular weight (Mw) of the functional group-free acrylic polymer is preferably 1000 to 9000, more preferably 1500 to 8000, and still more preferably 2000 to 6000, for effective reduction of the viscosity of the adhesive composition for skin and for preparation of an adhesive with high cohesive force. Here, Mw is a value in terms of polystyrene, as determined by gel-permeation chromatography (GPC).

The functional group-free acrylic polymer is preferably liquid at normal temperature (20 to 30°C) for effective reduction of the viscosity of the adhesive composition for skin. The functional group-free acrylic polymer liquid at room temperature preferably has a solid matter concentration of 90% or more, more preferably 95% or more, and still more preferably 98% or more.

The viscosity of the functional group-free acrylic polymer at 25°C is preferably 300 to 10000 mPa•s, more preferably 400 to 6000 mPa•s, and still more preferably 500 to 5000 mPa•s. In the present description, the viscosity (steady-flow viscosity) of the functional group-free acrylic polymer at 25°C is a value determined at 25°C, with a single-cylinder rotating viscometer according to JIS Z8803.

The functional group-free acrylic polymer preferably has a glass transition point (Tg) of -100 to -20°C, more preferably -90 to -40°C, and still more preferably -80 to -60°C for effective reduction of the viscosity of the adhesive composition for skin. Tg is a value determined by a differential scanning calorimeter (DSC).

The content of the functional group-free acrylic polymer is not particularly limited, but preferably 1 to 20 mass %, more preferably 5 to 18 mass %, and still more preferably 7.5 to 15 mass %, in the total mass of the adhesive composition for skin. When the content of the functional group-free acrylic polymer is in the range above, it is possible to reduce the viscosity of the adhesive composition for skin effectively and increase the cohesiveness of the adhesive effectively by using a reactive diluent described above simultaneously.

Before irradiation with radiation, the adhesive composition for skin in the embodiment described above preferably has a dynamic viscosity at 115°C and 1 Hz, as determined by the method described in Examples below, of 500 Pa•s or less, more preferably 300 Pa•s or less, and still more preferably 250 Pa•s or less.

When the viscosity of the adhesive composition for skin is in the range above, it is possible to process (for example coat) the adhesive composition for skin easily. It is possible, for example, to coat the adhesive composition on a hot-melt processing apparatus.

The adhesive composition for skin of the present embodiment is used as an adhesive for skin. The adhesive for skin is used favorably for adhesive sheets such as wound dressings, surgical tapes, tapes for fixing catheters and infusion tubes to the body, adhesive patches for ostomy device, plasters, tapes for fixing ECG electrodes and magnetic therapy apparatuses, and adhesive sheets for skin care and cosmetic purposes. In particular as described above, the adhesive composition for skin of the present embodiment is used favorably for wound dressings or adhesive patches for ostomy device, as it gives an adhesive for skin in the state of hydrocolloid. The adhesive for skin of the present embodiment, when used as a wound dressing, can absorb water such as sweat and wound exudate effectively and generate a wet environment suited for wound healing, thus improving wound-healing efficiency. The adhesive for skin of the present embodiment, when used as an adhesive patch for ostomy devices, can absorb water, for example, of sweat and excreta effectively and generate an environment less-incompatible or less-irritant to the skin.

As described above, as the adhesive composition for skin of the present embodiment contains a reactive diluent, it is less viscous and thus more readily coatable than the adhesive composition without a reactive diluent, before curing of the radiation-curable resin. As the radiation-curable resin cures and the reactive diluent is bound to the radiation-curable resin in the adhesive composition for skin by irradiation with radiation, it is possible to obtain an adhesive for skin having favorable cohesiveness after curing of the radiation-curable resin.

For preparation of a readily coatable and highly cohesive adhesive, the adhesive composition for skin preferably contains a radiation-curable resin described above in an amount of 30 to 95 mass %, a reactive diluent described above in an amount of 1 to 30 mass %, and additionally a functional group-free acrylic polymer in an amount of 1 to 20 mass %. For preparation of an adhesive more favorable to the skin, the adhesive composition for skin preferably contains a radiation-curable resin described above in an amount of 30 to 90 mass %, a reactive diluent described above in an amount of 1 to 30 mass %, a hydrophilic polymer described above in an amount of 5 to 20 mass %, and additionally a functional group-free acrylic polymer in an amount of 1 to 20 mass %.

It is possible to obtain a more favorable adhesive composition for skin by using the components in the amounts described in detail in the present embodiment.

<Adhesive for skin>

The adhesive for skin in the present embodiment of the invention, which is produced by irradiating the adhesive composition for skin of the present invention with radiation, is the adhesive composition for skin after irradiation with radiation.

The adhesive for skin of the present embodiment may be a fluidal liquid or in the solid state for example in a film or sheet shape. The adhesive for skin, independently of whether it is liquid or solid, can be used as an adhesive layer formed on a base sheet.

The irradiation amount of the radiation in the irradiation step of irradiating the adhesive composition for skin with radiation is not particularly limited, if it is an energy sufficient for causing curing reaction of the radiation-curable resin and also reaction of the reactive diluent with the radiation-curable resin.

For example when a UV-curable resin having a thickness of 100 $\mu$m is used as the radiation-curable resin, the integrated irradiation amount of an ultraviolet ray at a wavelength of 200 to 400 nm can be adjusted to an adequate value in the range of 10 to 1000 mJ/cm$^2$. It is preferably in the range of 100 to 400 mJ/cm$^2$.

Alternatively when an electron beam-curable resin is used as the radiation-curable resin, the integrated irradiation amount of the electron beam may be adjusted to a proper value, for example in the range of 1 to 100 kGy.

Preferably in production of the adhesive for skin of the present embodiment, there is a mixing step of mixing the adhesive composition for skin before the irradiation step.

The mixing method is not particularly limited, and, for example, the adhesive composition may be mixed, using a stirrer, a kneader, a roll, or the like. The period, temperature, and others of the mixing step are also not particularly limited and determined properly according to the components contained in the adhesive composition for skin according to the present invention.

In production of the adhesive for skin of the present embodiment, it is preferable, to make the curing reaction occur uniformly by irradiation with radiation, that there is a step of filling the adhesive composition for skin into a mold or applying it dropwise or coating it for example on a base sheet (hereinafter, referred to generally as "coating step") before the irradiation step.

The method of carrying out the coating step is not particularly limited and any known method may be used. A common method for example by means of comma coater, die coater, bar coater, knife coater, gravure roll, or reverse roll, or by spin coating, spray coating, screen printing, dipping, or dispensing may be used.

It is also preferable to provide it with air permeability during the coating. Specifically, it may be possible to form air channels at random in the adhesive composition, as a tension is applied to the adhesive composition by adjusting the clearance between the die coater head and the back roll. Another example is a method of forming air channels by applying an embossing roll to the surface of the adhesive composition and thus transferring the surface irregularity of the embossing roll onto the adhesive composition. Yet alternatively, the adhesive composition may be transferred by screen printing, by using a screen having holes previously formed with suitable gaps, making the adhesive composition pass through the holes and placing a base material on the rear face of the screen.

The adhesive composition for skin according to the present invention can also be applied on a hot-melt processing apparatus. In this case, when the easiness of coating operation and the heat resistance of the hydrophilic polymer in the adhesive composition for skin are taken into consideration, the coating temperature is preferably 60°C to 150°C, more preferably 70°C to 140°C, and still more preferably 80°C to 130°C.

<Adhesive sheet for skin>

The adhesive sheet for skin in the present embodiment of the present invention includes a base sheet and an adhesive layer formed thereon of the adhesive composition for skin of the present invention.

The shape of the adhesive sheet for skin of the present embodiment is not particularly limited, and may be, for example, polygonal (e.g., triangular, quadrangular, or rhomboid), circular, elliptic, or combination of the shapes above; a tape shape extending continuously in a particular direction, or a roll shape. The adhesive sheet for skin may be formed three-dimensionally according to the site to be adhered and thus may have cuts, slits, and the like.

The base sheet in the adhesive sheet for skin of the present embodiment supports the adhesive layer made of the adhesive composition for skin according to the present invention. The shape and the material of the base sheet are not particularly limited, if the base sheet can support the adhesive layer.

Examples of the base sheet include fibrous sheets such as nonwoven, knitted, and woven fabrics, plastic films, foam sheets, paper, and the like. Among the base sheets above, use of a plastic film is preferable from the viewpoints of flexibility, expandability/shrinkability, favorable steam permeability, and microbe-blocking efficiency.

Examples of the materials for the plastic film include polyurethanes; polyesters such as polyethylene terephthalate and polybutylene terephthalate; polyamides such as nylon 6 and nylon 66; polyolefins such as polyethylene and polypropylene; olefinic copolymers such as ethylene-vinyl acetate (EVA) copolymers and ethylene-alkyl (meth)acrylate-based copolymers; silicones such as polydimethylsiloxane and the like.

In the present embodiment, polyurethanes, polyesters, and polyamide, for example, are preferable among the plastic films above, as they are superior in steam permeability and do not inhibit insensitive perspiration or the like. These materials may be used alone or as a mixture of two or more.

The base sheet in the adhesive sheet for skin of the present embodiment may be made of a single material in a single shape, but may also be formed with two or more materials in a composite-like shape. Alternatively, a sheet in a laminate structure consisting of multiple base sheets in the same or different kinds of shape may be used as the base sheet.

The thickness of the base sheet in the adhesive sheet for skin of the present embodiment is not particularly limited, if it does not impair the object of the present invention, but preferably 1 to 100 $\mu$m and more preferably 5 to 50 $\mu$m, from the viewpoints of adhesiveness of the adhesive layer, easiness of application, and oppressive feeling of the skin.

The thickness of the adhesive layer in the adhesive sheet for skin of the present embodiment is not particularly limited, if it does not impair the object of the present invention, but preferably 0.001 to 10 mm and more preferably 0.02 to 2 mm. It is because such an adhesive sheet has favorable adhesive strength when it is applied and shows favorable adhesiveness and adaptability to the skin.

The adhesive sheet for skin of the present embodiment may include, in addition to the base sheet and the adhesive layer, a release sheet formed on the adhesive layer, as it faces the base sheet. The release sheet, when formed, protects the adhesive layer for example from contamination and makes the adhesive sheet for skin handled more easily.

The release sheet in the adhesive sheet for skin of the present embodiment may be made of any one of the materials traditionally used in the fields such as synthetic resin film, paper, and others. Examples thereof include papers and films surface-treated with a silicone or fluoroplastic resin, those embossed and the like.

A release sheet having high UV transmittance may be used as the release sheet in the adhesive sheet for skin of the present embodiment for acceleration of curing (or crosslinking) by UV irradiation. Examples of the release sheets having high UV transmittance for use include polypropylene sheets, polyethylene sheets, and the like. When such a release sheet is used, the adhesive sheet can be UV-irradiated from both faces for acceleration of curing (or crosslinking).

The adhesive sheet for skin of the present embodiment described above can be used in various applications including wound dressings, surgical tapes, tapes for fixing catheter, and the like. In particular, the adhesive sheet for skin of the present embodiment can improve the wound healing efficiency drastically, when used in the wound healing application.

The adhesive sheet for skin of the present embodiment can be prepared by carrying out at least a coating step of applying dropwise or coating the adhesive composition for skin of the present invention on the surface of the base sheet and an irradiation step of curing the adhesive composition for skin by irradiation thereof with radiation. The methods of carrying out the coating and irradiation steps may be methods similar to those described in the section of "Adhesive for skin" above.

Examples

Hereinafter, the present invention will be described more in detail with reference to Examples. The Examples described below are some of the typical examples of present invention and it should be understood that the scope of the present invention is not restricted thereby.

<Example 1>

A UV-curable resin as an example of the radiation-curable resin (trade name: "acResin A260UV," manufactured by BASF, a polymer having a butyl acrylate-derived structural unit) in an amount of 90.0 mass % was heated to 120°C; a PO-modified trimethylolpropane triacrylate as an example of the reactive diluent (trade name "TMP-3P", manufactured by Dai-ichi Kogyo Seiyaku Co., Ltd., viscosity at 25°C: 60 mPa•s) in an amount of 10.0 mass % was added thereto; and the mixture was mixed and agitated. The mixture was then stored at a temperature at which the viscosity thereof became 150 Pa•s or less, to give an adhesive composition for skin of Example 1.

The adhesive composition for skin obtained was then applied on a PET film as an example of the base sheet (thickness: 25 $\mu$m) to a thickness of 100 $\mu$m, using a comma coater under a condition of 120°C. Ultraviolet ray was irradiated to the adhesive composition for skin coated on the PET film to an integrated light intensity of 200 mJ/cm$^2$ on a UV-irradiating apparatus, to give an adhesive sheet for skin having an adhesive for skin formed on the PET film.

<Example 2>

A UV-curable resin as an example of the radiation-curable resin (trade name: "acResin A260UV," manufactured by BASF) in an amount of 70.0 mass % and a rosin derivative as an example of tackifier (manufactured by Arakawa Chemical Industries, Ltd.) in an amount of 7.5 mass % were mixed and heated to 120°C.

The PO-modified trimethylolpropane triacrylate used in Example 1 as an example of the reactive diluent was added thereto in an amount of 7.5 mass %; the mixture was mixed and agitated, and then stored at a temperature at which the viscosity thereof became 150 Pa•s or less.

Then, sodium carboxymethylcellulose as an example of the hydrophilic polymer (hereinafter, "CMC-Na," manufactured by Nippon Paper Industries Co., Ltd.) in an amount of 14.0 mass % and silica as an example of the filler (manufactured by Tosoh Silica Corporation) in an amount of 1.0 mass % were added thereto and the mixture was agitated, to give an adhesive composition for skin of Example 2. An adhesive for skin and an adhesive sheet for skin of Example 2 were prepared, using the adhesive composition for skin, similarly to Example 1.

<Example 3>

An adhesive composition for skin of Example 3 was prepared in a manner similar to Example 2, except that the reactive diluent in the adhesive composition for skin of Example 2 was replaced with polyethylene glycol diacrylate as an example thereof (trade name "PE300", manufactured by Dai-ichi Kogyo Seiyaku Co., Ltd., viscosity at 25°C: 32 mPa•s, number of ethylene glycol constituent units: approximately 6) in an amount of 7.5 mass %. An adhesive for skin and an adhesive sheet for skin of Example 3 were prepared, using the adhesive composition for skin, similarly to Example 1.

<Example 4>

An adhesive composition for skin of Example 4 was prepared in a manner similar to Example 2, except that the reactive diluent in the adhesive composition for skin of Example 2 was replaced with N-acryloylmorpholine as an example thereof (trade name "ACMO," manufactured by Kohjin Film & Chemicals Co., Ltd., viscosity at 25°C: 12 mPa•s) in an amount of 7.5 mass %. An adhesive for skin and an adhesive sheet for skin of Example 4 were prepared, using the adhesive composition for skin, similarly to Example 1.

<Example 5>

An adhesive composition for skin of Example 5 was prepared in a manner similar to Example 2, except that the contents of the UV-curable resin and the reactive diluent in adhesive composition for skin of Example 2 were respectively changed to 62.5 mass % and 15.0 mass %. An adhesive for skin and an adhesive sheet for skin of Example 5 were prepared, using the adhesive composition for skin, similarly to Example 1.

<Example 6>

An adhesive composition for skin of Example 6 was prepared in a manner similar to Example 2, except that the contents of the UV-curable resin and the reactive diluent in the adhesive composition for skin of Example 2 were changed respectively to 47.5 mass % and 30.0 mass %. An adhesive for skin and an adhesive sheet for skin of Example 6 were prepared, using the adhesive composition for skin, similarly to Example 1.

<Example 7>

An UV-curable resin as an example of radiation-curable resin (trade name "acResin A260UV," manufactured by BASF) was used in an amount of 72.5 mass % in the entire composition and the mixture was heated to 120°C.

The PO-modified trimethylolpropane triacrylate used in Example 1 as an example of reactive diluent was added thereto in an amount of 2.5 mass % in the entire composition; a functional group-free acrylic polymer A liquid at 25°C (trade name "ARUFON® UP-1000, manufactured by Toagosei Co., Ltd.," Mw: 3000, viscosity at 25°C: 1000 mPa•s, Tg: -77°C, solid matter concentration: 98% or more) was added thereto in an amount of 10.0 mass % in the entire composition; the mixture was mixed and agitated, and then stored at a temperature at which the viscosity was 150 Pa•s or less.

Then, the CMC-Na used in Example 2 was added thereto as an example of hydrophilic polymer in an amount of 14.0 mass % in the entire composition; the silica used in Example 2 (manufactured by Tosoh Silica Corporation) was also added thereto as an example of filler in an amount of 1.0 mass % in the entire composition; and the mixture was agitated, to give an adhesive composition for skin of Example 7. An adhesive for skin and an adhesive sheet for skin of Example7 were prepared, using the adhesive composition for skin, similarly to Example 1.

<Example 8>

An adhesive composition for skin of Example 8 was prepared in a manner similar to Example 7, except that the contents of the reactive diluent and the acrylic polymer A in the adhesive composition for skin of Example 7 were respectively changed to 1.0 mass % and 11.5 mass%. An adhesive for skin and an adhesive sheet for skin of Example 8 were prepared, using the adhesive composition for skin, similarly to Example 1.

<Example 9>

An adhesive composition for skin of Example 9 was prepared in a manner similar to Example 7, except that the contents of the UV-curable resin, the reactive diluent, and the acrylic polymer A in the adhesive composition for skin of Example 7 were respectively changed to 62.5 mass %, 7.5 mass %, and 15.0 mass %. An adhesive for skin and an adhesive sheet for skin of Example 9 were prepared, using the adhesive composition for skin, similarly to Example 1.

<Example 10>

An adhesive composition for skin of Example 10 was prepared in a manner similar to Example 7, except that the contents of the UV-curable resin, the reactive diluent, and the acrylic polymer A in the adhesive composition for skin of Example 7 were respectively changed to 62.5 mass %, 15.0 mass %, and 7.5 mass %. An adhesive for skin and an adhesive sheet for skin of Example 10 were prepared, using the adhesive composition for skin, similarly to Example 1.

<Example 11>

An adhesive composition for skin of Example 11 was prepared in a manner similar to Example 7, except that the acrylic polymer A in the adhesive composition for skin of Example 7 was replaced with a functional group-free acrylic polymer B that is liquid at 25°C (trade name: "ARUFON® UP-1020," manufactured by Toagosei Co., Ltd., Mw: 2000, viscosity at 25°C: 500 mPa•s, Tg: -80°C, solid matter concentration: 98% or more). An adhesive for skin and an adhesive sheet for skin of Example 11 were prepared, using the adhesive composition for skin, similarly to Example 1.

<Example 12>

An adhesive composition for skin of Example 12 was prepared in a manner similar to Example 7, except that the acrylic polymer A in the adhesive composition for skin of Example 7 was replaced with a functional group-free acrylic polymer C that is liquid at 25°C (trade name "ARUFON® UP-1080," manufactured by Toagosei Co., Ltd., Mw: 6000, viscosity at 25°C: 5000 mPa•s, Tg: -61°C, solid matter concentration: 98% or more). An adhesive for skin and an adhesive sheet for skin of Example 12 were prepared, using the adhesive composition for skin, similarly to Example 1.

<Comparative Example 1>

An adhesive composition for skin of Comparative Example 1 was prepared in a manner similar to Example 1, except that the reactive diluent in the adhesive composition for skin of Example 1 was replaced with bis[2-(2-butoxyethoxy)ethyl] adipate as an example of plasticizer (trade name "TP-95," manufactured by Morton International Inc.) in an amount of 10.0 mass %. An adhesive for skin and an adhesive sheet for skin of Comparative Example 1 were prepared, using the adhesive composition for skin, similarly to Example 1.

<Comparative Example 2>

An adhesive composition for skin of Comparative Example 2 was prepared in a manner similar to Example 1, except that the reactive diluent in the adhesive composition for skin of Example 1 was replaced with tris(2-ethylhexyl) trimellitate as an example of plasticizer (manufactured by Daihachi Chemical Industry Co., Ltd.) in an amount of 10.0 mass %. An adhesive for skin and an adhesive sheet for skin of Comparative Example 2 were prepared, using the adhesive composition for skin, similarly to Example 1.

<Comparative Example 3>

An adhesive composition of Comparative Example 3 was prepared in a manner similar to Example 2, except that the reactive diluent in the adhesive composition for skin of Example 2 was replaced with the acrylic polymer A used in Example 7 in an amount of 7.5 mass %. An adhesive and an adhesive sheet of Comparative Example 3 were prepared, using the adhesive composition, similarly to Example 1.

<Comparative Example 4>

An adhesive composition of Comparative Example 4 was prepared in a manner similar to Example 2, except that the reactive diluent in the adhesive composition for skin of Example 2 was replaced with bis[2-(2-butoxyethoxy)ethyl] adipate as an example of plasticizer (trade name "TP-95," manufactured by Morton International Inc.) in an amount of 7.5 mass %. An adhesive and an adhesive sheet of Comparative Example 4 were prepared, using the adhesive composition, similarly to Example 1.

<Comparative Example 5>

An adhesive composition of Comparative Example 5 was prepared in a manner similar to Example 2, except that the reactive diluent in the adhesive composition for skin of Example 2 was replaced with polyoxyalkylene monobutylether as an example of plasticizer (trade name: "50BH-100," manufactured by Sanyo Chemical Industries, Ltd.) in an amount of 7.5 mass %. An adhesive and an adhesive sheet of Comparative Example 5 were prepared, using the adhesive composition, similarly to Example 1.

<Comparative Example 6>

An adhesive composition of Comparative Example 6 was prepared in a manner similar to Example 2, except that the reactive diluent in the adhesive composition for skin of Example 2 was replaced with tris(2-ethylhexyl) trimellitate as an example of plasticizer (manufactured by Daihachi Chemical Industry Co., Ltd.) in an amount of 7.5 mass %. An adhesive and an adhesive sheet of Comparative Example 6 were prepared, using the adhesive composition, similarly to Example 1.

<Comparative Example 7>

An adhesive composition of Comparative Example 7 was prepared in a manner similar to Example 2, except that the reactive diluent in the adhesive composition for skin of Example 2 was replaced with di(2-ethylhexyl) sebacate as an example of plasticizer (manufactured by New Japan Chemical Co., Ltd.) in an amount of 15.0 mass %. An adhesive and an adhesive sheet of Comparative Example 7 were prepared, using the adhesive composition, similarly to Example 1.
The dynamic viscosity of the adhesive compositions of Examples and Comparative Examples above was determined by the following method.

<Dynamic viscosity>

The dynamic viscosity (dynamic complex viscosity) of each adhesive composition was determined under the condition of a measurement temperature of 20 to 120°C, a measurement thickness of 1 mm, a measurement pressure of 100 Pa, and a measurement frequency of 1 Hz, using a dynamic viscoelastometer (product name: "RS 150 RheoStress," manufactured by HAAKE). The dynamic viscosity at 115°C is shown in the Table below.

(Viscosity decrease rate)

In addition, the viscosity decrease rate was calculated based on the test results of dynamic viscosity at 115°C according to the formula below. The dynamic viscosity of "standard adhesive composition" in the following formula was the dynamic viscosity of the UV-curable resin (trade name: "acResin A260UV," manufactured by BASF) at 115°C (dynamic viscosity: 218.9 Pa•s) in the cases of Example 1 and Comparative Examples 1 and 2, and the dynamic viscosity of the adhesive composition of Comparative Example 3 at 115°C (dynamic viscosity: 286.5 Pa•s) in the cases of Examples 2 to 12 and Comparative Examples 3 to 7.

[Formula 1]

$$\text{Viscosity decrease rate} = \left\{ 1 - \frac{\text{Dynamic viscosity of the adhesive composition of each Example or Comparative Example}}{\text{Dynamic viscosity of standard adhesive composition}} \right\} \times 100$$

The adhesive strength (peel strength) of the adhesive of each of the adhesive compositions of Examples and Comparative Examples above was determined according to JIS Z0237, using a tensile tester (product name: "INSTRON5564," produced by Instron) at a stress rate of 300 mm/min. The test piece for measurement was prepared by applying each adhesive on a Bakelite plate as the substrate. The adhesive was formed by irradiating ultraviolet ray similarly to the production condition described above and the size of the test piece was 1 inch (25.4 mm) in width and 100 mm in length.

In measurement of the adhesive strength, it was examined whether the mode of the adhesive being separated from the substrate in each test piece (exfoliation mode) is "interfacial separation," wherein the adhesive was separated from the substrate at the interface, or "cohesive failure," wherein the adhesive was broken down.

The compositions of the adhesive compositions of Examples and Comparative Examples and the test results of the dynamic viscosity and the adhesive strength thereof are summarized in Tables 1 to 4. "-" in the column of the composition in Tables 1 to 4 indicates that the component was not contained.

[Table 1]

|  | Example 1 | Comparative Example 1 | Comparative Example 2 |
|---|---|---|---|
| UV-curable resin (mass %) | 90.0 | 90.0 | 90.0 |
| PO-modified TMPTA (mass %) | 10.0 | - | - |
| TP-95 (mass %) | - | 10.0 | - |
| TOTM (mass %) | - | - | 10.0 |
| Dynamic viscosity (Pa•s at 115°C) | 126.2 | 110.0 | 145.0 |
| Viscosity decrease rate (%) | 42.3 | 49.7 | 33.8 |
| Adhesive strength (N/inch) | 11.25 | 10.55 | 18.30 |
| Exfoliation mode | Interfacial separation | Cohesive failure | Cohesive failure |
| •PO-modified TMPTA: PO-modified trimethylolpropane triacrylate<br>•TP-95: bis[2-(2-butoxyethoxy)ethyl] adipate<br>•TOTM: tris(2-ethylhexyl) trimellitate | | | |

[Table 2]

|  | Example 2 | Example 3 | Example 4 | Example 5 | Example 6 | Comparative Example 3 |
|---|---|---|---|---|---|---|
| UV-curable resin (mass %) | 70.0 | 70.0 | 70.0 | 62.5 | 47.5 | 70.0 |
| Acrylic polymer (mass %) | - | - | - | - | - | 7.5 |

(continued)

|  | Example 2 | Example 3 | Example 4 | Example 5 | Example 6 | Comparative Example 3 |
|---|---|---|---|---|---|---|
| PO-modified TMPTA (mass %) | 7.5 | - | - | 15.0 | 30.0 | - |
| PEGDA (mass %) | - | 7.5 | - | - | - | - |
| ACMO (mass %) | - | - | 7.5 | - | - | - |
| Rosin derivative (mass %) | 7.5 | 7.5 | 7.5 | 7.5 | 7.5 | 7.5 |
| CMC-Na (mass %) | 14.0 | 14.0 | 14.0 | 14.0 | 14.0 | 14.0 |
| Silica (mass %) | 1.0 | 1.0 | 1.0 | 1.0 | 1.0 | 1.0 |
| Dynamic viscosity (Pa•s at 115°C) | 203.4 | 177.8 | 191.3 | 107.7 | 22.53 | 286.5 |
| Viscosity decrease rate (%) | 29.0 | 37.9 | 33.2 | 62.4 | 92.1 | 0 |
| Adhesive strength (N/inch) | 13.1 | 14.8 | 10.6 | 2.9 | 2.2 | 20.3 |
| Exfoliation mode | Interfacial separation | Interfacial separation | Interfacial separation | Interfacial separation | Interfacial separation | Interfacial separation |
| • PO-modified TMPTA: PO-modified trimethylolpropane triacrylate<br>• PEGDA: polyethylene glycol diacrylate<br>• ACMO: N-acryloylmorpholine | | | | | | |

[Table 3]

|  | Comparative Example 4 | Comparative Example 5 | Comparative Example 6 | Comparative Example 7 |
|---|---|---|---|---|
| UV-curable resin (mass %) | 70.0 | 70.0 | 70.0 | 62.5 |
| TP-95 (mass %) | 7.5 | - | - | - |
| 50BH-100 (mass %) | - | 7.5 | - | - |
| TOTM (mass %) | - | - | 7.5 | - |
| DOS (mass %) | - | - | - | 15.0 |
| Rosin derivative (mass %) | 7.5 | 7.5 | 7.5 | 7.5 |
| CMC-Na (mass %) | 14.0 | 14.0 | 14.0 | 14.0 |
| Silica (mass %) | 1.0 | 1.0 | 1.0 | 1.0 |

(continued)

|  | Comparative Example 4 | Comparative Example 5 | Comparative Example 6 | Comparative Example 7 |
|---|---|---|---|---|
| Dynamic viscosity (Pa•s at 115°C) | 218.9 | 172.7 | 259.8 | 77.9 |
| Viscosity decrease rate (%) | 23.6 | 39.7 | 9.3 | 72.8 |
| Adhesive strength (N/inch) | 24.3 | 14.1 | 19.12 | 10.3 |
| Exfoliation mode | Cohesive failure | Cohesive failure | Interfacial separation | Cohesive failure |
| • TP-95: bis[2-(2-butoxyethoxy)ethyl] adipate<br>• 50BH-100: polyoxyalkylene monobutylether<br>• TOTM: tris(2-ethylhexyl) trimellitate<br>• DOS: di(2-ethylhexyl) sebacate | | | | |

[Table 4]

|  | Example 7 | Example 8 | Example 9 | Example 10 | Example 11 | Example 12 |
|---|---|---|---|---|---|---|
| UV-curable resin (mass %) | 72.5 | 72.5 | 62.5 | 62.5 | 72.5 | 72.5 |
| Acrylic polymer A (mass %) | 10.0 | 11.5 | 15.0 | 7.5 | - | - |
| Acrylic polymer B (mass %) | - | - | - | - | 10.0 | - |
| Acrylic polymer C (mass %) | - | - | - | - | - | 10.0 |
| PO-modified TMPTA (mass %) | 2.5 | 1.0 | 7.5 | 15.0 | 2.5 | 2.5 |
| Rosin derivative (mass %) | - | - | - | - | - | - |
| CMC-Na (mass %) | 14.0 | 14.0 | 14.0 | 14.0 | 14.0 | 14.0 |
| Silica (mass %) | 1.0 | 1.0 | 1.0 | 1.0 | 1.0 | 1.0 |
| Dynamic viscosity (Pa•s at 115°C) | 137.0 | 193.9 | 96.0 | 68.1 | 189.1 | 213.2 |
| Viscosity decrease rate (%) | 52.2 | 32.3 | 66.5 | 76.2 | 34.0 | 25.6 |
| Adhesive strength (N/inch) | 16.8 | 19.2 | 10.3 | 8.6 | 16.3 | 16.7 |
| Exfoliation mode | Interfacial separation | Interfacial separation | Interfacial separation | Interfacial separation | Interfacial separation | Interfacial separation |
| •Acrylic polymer A: functional group-free acrylic polymer having a Mw of 3000, a viscosity at 25°C of 1000 mPa•s, and a Tg of -77°C<br>•Acrylic polymer B: functional group-free acrylic polymer having a Mw of 2000, a viscosity at 25°C of 500 mPa•s, and a Tg of -80°C | | | | | | |

(continued)

|  | Example 7 | Example 8 | Example 9 | Example 10 | Example 11 | Example 12 |
|---|---|---|---|---|---|---|
| •Acrylic polymer C: functional group-free acrylic polymer having a Mw of 6000, a viscosity at 25°C of 5000 mPa•s, and a Tg of -61°C<br>• PO-modified TMPTA: PO-modified trimethylolpropane triacrylate | | | | | | |

As shown in Table 1, the adhesive compositions for skin of Example 1 and Comparative Examples 1 and 2 were found to have a viscosity lower than that of their pure UV-curable resins. However, the adhesives of Comparative Examples 1 and 2 were found to be broken down (cohesive failure) during measurement of the adhesive strength and were thus inferior in cohesiveness. The result that the adhesive for skin of Example 1 has lower viscosity and yet does not show cohesive failure suggests that it has favorable cohesiveness.

As shown in Table 2, the adhesive compositions for skin of Examples 2 to 6, which contain a reactive diluent, had a viscosity effectively lower than that of the adhesive composition for skin of Comparative Example 3 and could be coated easily. In addition, the adhesives for skin of Examples 2 to 6 showed favorable adhesive strength and did not cause cohesive failure. Further, increase in the amount of the reactive diluent led to reduction in viscosity without cohesive failure. The results suggest that the adhesives for skin of Examples 2 to 6 have favorable cohesiveness.

As shown in Table 3, the adhesive compositions of Comparative Examples 4 to 7, which contained a plasticizer, had a viscosity lower than that of the adhesive composition for skin of Comparative Example 3. However, the adhesive of Comparative Example 6 showed only small decrease in viscosity. In addition, the adhesives of Comparative Examples 4, 5, and 7 were found to be broken down (cohesive failure) during measurement of adhesive strength and were thus inferior in cohesiveness. Further as shown in Comparative Example 7, increase in the amount of the plasticizer led not only to decrease in viscosity but also to cohesive failure, making the adhesive practically unusable.

As shown in Table 4, the adhesive compositions for skin of Examples 7 to 12, which included both a reactive diluent and a functional group-free acrylic polymer, were effectively lower in viscosity and showed favorable cohesiveness.

The adhesive compositions for skin of Examples 7 to 9, 11, and 12, which had a reactive diluent content in the range of 1.0 to 7.5 mass % and a functional group-free acrylic polymer content of in the range of 10.0 to 15.0 mass %, had effectively lower viscosity and showed a high adhesive strength of more than 10 N/inch.

As obvious from Tables 1 to 4, the adhesive composition for skin according to the present invention, which is less viscous and yet resistant to cohesive failure, has an advantageous effect that a hydrophilic polymer may be mixed without use of a high-viscosity mixer such as pressure kneader. In addition, as the adhesive composition for skin according to the present invention is less viscous, it has an advantageous effects that it can be coated more easily than ever.

**Claims**

1. An adhesive composition for skin, comprising:

   a radiation-curable resin; and
   a reactive diluent that reacts with the radiation-curable resin.

2. The adhesive composition for skin according to Claim 1, wherein the radiation-curable resin is a UV-curable resin.

3. The adhesive composition for skin according to Claim 1 or 2, wherein the reactive diluent comprises a (meth)acryloyl group-containing compound.

4. The adhesive composition for skin according to any one of Claims 1 to 3, comprising:

   a UV-curable resin as the radiation-curable resin in an amount of 30 to 95 mass %; and
   a (meth)acryloyl group-containing reactive diluent as the reactive diluent in an amount of 1 to 30 mass %.

5. The adhesive composition for skin according to any one of Claims 1 to 4, wherein the reactive diluent is a reactive diluent comprising 1 to 3 (meth)acryloyl groups in the molecule.

6. The adhesive composition for skin according to any one of Claims 1 to 5, wherein the reactive diluent is a trimethylolpropane skeleton-containing (meth)acrylate.

7. The adhesive composition for skin according to any one of Claims 1 to 6, further comprising a hydrophilic polymer.

8. The adhesive composition for skin according to any one of Claims 1 to 7, comprising:

a UV-curable resin as the radiation-curable resin in an amount of 30 to 90 mass %;
the reactive diluent in an amount of 1 to 30 mass %; and
a hydrophilic polymer in an amount of 5 to 20 mass %.

9. The adhesive composition for skin according to any one of Claims 1 to 8, further comprising a functional group-free acrylic polymer.

10. The adhesive composition for skin according to any one of Claims 1 to 9, further comprising a functional group-free acrylic polymer in an amount of 1 to 20 mass %.

11. An adhesive for skin, prepared by irradiating the adhesive composition for skin according to any one of Claims 1 to 10 with radiation.

12. An adhesive sheet for skin, comprising a base sheet and an adhesive layer formed thereon that is prepared by irradiating the adhesive composition for skin according to any one of Claims 1 to 10 with radiation.

**EP 2 924 092 A1**

**INTERNATIONAL SEARCH REPORT**

International application No.

PCT/JP2013/081316

A. CLASSIFICATION OF SUBJECT MATTER
*C09J201/00*(2006.01)i, *A61K9/70*(2006.01)i, *C09J4/02*(2006.01)i, *C09J7/02*
(2006.01)i, *C09J11/06*(2006.01)i, *C09J11/08*(2006.01)i

According to International Patent Classification (IPC) or to both national classification and IPC

B. FIELDS SEARCHED

Minimum documentation searched (classification system followed by classification symbols)
C09J201/00, A61K9/70, C09J4/02, C09J7/02, C09J11/06, C09J11/08

Documentation searched other than minimum documentation to the extent that such documents are included in the fields searched
Jitsuyo Shinan Koho          1922-1996   Jitsuyo Shinan Toroku Koho   1996-2014
Kokai Jitsuyo Shinan Koho   1971-2014   Toroku Jitsuyo Shinan Koho   1994-2014

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2002-348543 A  (Lintec Corp.),<br>04 December 2002 (04.12.2002),<br>claims; paragraphs [0043], [0046] to [0048];<br>table 1<br>(Family: none) | 1-6,11-12<br>7-8<br>9-10 |
| X<br>Y<br>A | JP 57-14527 A  (Nitto Electric Industrial Co.,<br>Ltd.),<br>25 January 1982 (25.01.1982),<br>page 1, lower left column, 2. 'claims'; page 4,<br>upper left column, examples<br>(Family: none) | 1,11-12<br>7-8<br>2-6,9-10 |

[X] Further documents are listed in the continuation of Box C.   [ ] See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>19 February, 2014 (19.02.14) | Date of mailing of the international search report<br>04 March, 2014 (04.03.14) |
|---|---|
| Name and mailing address of the ISA/<br>Japanese Patent Office | Authorized officer |
| Facsimile No. | Telephone No. |

Form PCT/ISA/210 (second sheet) (July 2009)

19

INTERNATIONAL SEARCH REPORT

International application No.

PCT/JP2013/081316

C (Continuation).    DOCUMENTS CONSIDERED TO BE RELEVANT

| Category* | Citation of document, with indication, where appropriate, of the relevant passages | Relevant to claim No. |
|---|---|---|
| X<br>Y<br>A | JP 56-108707 A  (Nitto Electric Industrial Co.,<br>Ltd.),<br>28 August 1981 (28.08.1981),<br>page 1, lower left column, 2. 'claims'; page 5,<br>upper left column, example 1<br>(Family: none) | 1,11-12<br>7-8<br>2-6,9-10 |
| X<br>A | JP 2012-503698 A  (Lumina Adhesives AB),<br>09 February 2012 (09.02.2012),<br>claims; paragraphs [0081] to [0088]; table 2<br>& US 2011/0224593 A1     & GB 904582 D<br>& GB 904582 D0            & EP 2344602 A1<br>& WO 2010/034998 A1       & AU 2009295620 A<br>& CA 2739168 A            & CN 102224214 A<br>& NZ 592318 A | 1-2<br>3-12 |
| X<br>A | JP 1-240589 A  (The Furukawa Electric Co.,<br>Ltd.),<br>26 September 1989 (26.09.1989),<br>page 3, upper left column, examples<br>(Family: none) | 1-2<br>3-12 |
| Y<br>A | JP 2011-182847 A  (3M Innovative Properties<br>Co.),<br>22 September 2011 (22.09.2011),<br>claim 1; paragraphs [0028] to [0030], [0046];<br>paragraph [0051], table 1<br>& EP 2542636 A1            & WO 2011/109672 A1<br>& CN 102844395 A | 7-8<br>1-6,9-12 |
| Y<br>A | JP 59-232553 A  (Nitto Electric Industrial Co.,<br>Ltd.),<br>27 December 1984 (27.12.1984),<br>page 1, lower left column, 1. Title of the<br>Invention, 2. 'claims'<br>(Family: none) | 7-8<br>1-6,9-12 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000503009 T